(19) Europäisches Patentamt
European Patent Office
Office européen des brevets

(11) EP 4 470 489 A1

(12) **EUROPEAN PATENT APPLICATION**
published in accordance with Art. 153(4) EPC

(43) Date of publication:
04.12.2024 Bulletin 2024/49

(51) International Patent Classification (IPC):
A61B 18/12 (2006.01)        A61B 18/14 (2006.01)
A61B 90/00 (2016.01)

(21) Application number: 22923365.5

(22) Date of filing: 31.10.2022

(86) International application number:
PCT/CN2022/128641

(87) International publication number:
WO 2023/142567 (03.08.2023 Gazette 2023/31)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 27.01.2022 CN 202210102213

(71) Applicant: Sichuan Jinjiang Electronic Medical
Device
Technology Co., Ltd.
Chengdu, Sichuan 610045 (CN)

(72) Inventors:
• ZHU, Xiaolin
Chengdu, Sichuan 610045 (CN)
• SHI, Tiancai
Chengdu, Sichuan 610045 (CN)

(74) Representative: Lermer, Christoph
Nove Haus - Design Offices
Luise-Ullrich-Straße 14
80636 München (DE)

(54) **ABLATION CATHETER**

(57) An ablation catheter, having an annular distal end (1). The annular distal end (1) comprises an arc-shaped tail end catheter body (3). In the ablation catheter, a plurality of electrode pairs (2) are arranged at intervals along the arc-shaped extending direction of the tail end catheter body (3). The plurality of electrode pairs (2) are respectively wound on the tail end catheter body (3). Each electrode pair (2) comprises two ring electrodes (11, 12). Each ring electrode (11, 12) is wound on the tail end catheter body (3). According to the design of the electrode pairs, on the premise of ensuring the ablation effectiveness and safety, the diameter of the annular distal end (1) is more convenient to adjust. Moreover, the electrode pairs (2) are used for collecting electrophysiological signals more accurately, and the situation that the change determination of electrical signals is affected by the large distance between the electrodes is avoided.

Fig 2

EP 4 470 489 A1

## Description

## Technical Field

The present invention relates to the field of electrophysiological ablation, and more particularly to an ablation catheter having an annular distal end with an adjustable diameter.

## Background Art

Currently, the radiofrequency (RF) ablation and cryoablation are commonly used in the treatment of atrial fibrillation and other arrhythmias. The success of ablation depends primarily on the quality and adequacy of the lesions produced during the procedure. The injury must be sufficient to destroy arrhythmogenic tissue or sufficiently interfere with or isolate abnormal electrical conduction within the myocardial tissue. However, excessive ablation will affect surrounding healthy tissues as well as neural tissues. The disadvantages of radiofrequency ablation are that the ablation operation time is long and the operator is required to have a high level of catheter manipulation. The ablation is accompanied by pain due to thermal injury, and the postoperative pulmonary vein stenosis is easy to occur. The application of RF energy to the target tissue has an effect on a non-target tissue. The application of RF energy to the atrial wall tissue may cause damage to the esophagus or phrenic nerve. Further, RF ablation has the risk of tissue scabbing, further leading to embolic problems. However, for cryoablation, if the cryoballoon is closely attached to the pulmonary vein, the circular ablation and the isolation can be completed once or several times, and the patient does not have pain and shorten the operation time, but the cryoablation has a high incidence of phrenic nerve injury.

The application of a brief high voltage to the tissue using pulsed electric field techniques can produce a local high electric field of several hundred volts per centimeter that destroys the cell membrane by creating pores in the cell membrane (the cell membrane becomes "permeable"). Since different tissue cells have different thresholds for voltage penetration, the use of pulsed electric field technology can selectively treat myocardial cells (with relatively low thresholds) without affecting other non-target cell tissues (such as nerves, esophagus, blood vessels and blood cells). Meanwhile, the pulse technique will not produce a thermal effect due to the very short energy release time, thus avoiding the problems of tissue scab, pulmonary vein stenosis and so on.

However, the voltage of high-voltage pulse is relatively high, the energy between electrodes cannot be concentrated too much, and safety accidents are prone to occur, so that it is necessary to strengthen the electrode insulation and the internal insulation of catheter. If the existing ablation catheter uses high-voltage pulse to perform ablation, the ionization is easy to occur between electrodes, and the ablation time using high-voltage pulse is short, which requires more precise positioning.

The atrial fibrillation (AF) is a common sustained arrhythmia that seriously endangers human health and affects quality of life. Pulmonary veins are the most common focal lesions of atrial fibrillation because of the presence of pulmonary vein muscle sleeves. There are clusters of cardiomyocytes between the intima and adventitia of the pulmonary veins, which are surrounded by sleeves from the atrial side to the pulmonary side, called myocardial sleeves. Because the cells forming the myocardial sleeve are of different origins and electrophysiological properties from the atrial muscles, an abnormally excited matrix is formed. The atrial muscles surrounding the pulmonary veins, like the pulmonary veins, also contain a trigger focus for AF or an arrhythmic matrix that maintains AF, so that myocardial tissue surrounding the pulmonary veins is sometimes ablated during ablation isolation.

At present, the common method is point-by-point ablation of pulmonary vein antrum to form an annular isolation band. This method has long operation time, brings great pressure to patients and doctors, and often has leakage point to cause recurrence. Therefore, it is urgent to design a catheter which can rapidly ablate and isolate the pulmonary vein antrum at one time.

The currently known ring electrode catheter is in the form of fixed shape and size, which cannot achieve good contact and manipulation for the structural tissues with different cavity sizes.

Accordingly, there is a need for an ablation catheter having an annular distal end with adjustable diameter properties to accommodate different tissue structures and good abutment to form a closed annular ablation zone upon ablation discharge.

## Summary of the Invention

The present invention provides an ablation catheter having an annular distal end. On the one hand, the diameter of the annular distal end may be adjusted in order to adapt to different tissue structures and achieve a good abutment. On the other hand, an electrode pair is disposed on the annular distal end to accommodate deformation contraction caused by diameter adjustment of the annular distal end. Furthermore, the ring electrodes of the electrode pairs act in pairs in the ablation mode and individually in the mapping mode.

According to a first aspect of the present invention, an ablation catheter is provided. The ablation catheter having an annular distal end comprising an arc-shaped tail end catheter body. A plurality of electrode pairs are arranged at intervals along the arc-shaped extending direction of the tail end catheter body, and the plurality of electrode pairs are respectively wound on the tail end catheter body.

Preferably, the number of electrode pairs is 2N+1, where N is a positive integer.

In the ablation catheter according to the first aspect of the invention, each electrode pair comprises two ring electrodes surrounding the tail end catheter body; the width of each ring electrode in the arc-shaped extending direction of the tail end catheter body is L; the spacing between two ring electrodes in the same electrode pair is d; the spacing between adjacent electrode pairs is D; and the diameter of each ring electrode is S, then S>D=(2×L+d)×k, where k is a correction factor and k ranges from 0.7 to 1.4.

Preferably, L has a value of 0.50-1.5 mm; d has a value of 1-3 mm; and D has a value of 3-6 mm.

Preferably, the ring electrode may be a spiral electrode. The spiral electrode may be formed by winding an electrode wire, or the ring electrode may be cut into an electrode having a spiral shape.

Preferably, the catheter is a pulsed electric field ablation catheter for delivering and releasing pulsed ablation energy to a desired ablation site.

Preferably, the catheter is used for ablation of the heart and surrounding tissues.

When used for the pulsed electric field ablation, the two electrodes of each electrode pair are of the same polarity, equivalent to applying a voltage to the same electrode, and adjacent electrode pairs are of opposite polarity.

The two electrodes of each electrode pair are each independently configured for acquiring electrophysiological signals when used for electrophysiological mapping.

Preferably, the voltage amplitude applied by each electrode pair is 1000-4000V

In the ablation catheter according to the first aspect of the invention, a positioning sensor is arranged below the electrode pair, inside the tail end catheter body.

Preferably, the length of the positioning sensor in the arc-shaped extension of the tail end catheter body is equal to the width of the electrode pair in the arc-shaped extension of the tail end catheter body. Specifically, the length of the positioning sensor in the direction of the arc-shaped extension of the tail end catheter body is equal to the sum of the width of the two ring electrodes of the electrode pair respectively in the direction of the arc-shaped extension of the tail end catheter body and their spacing.

Preferably, the positioning sensor comprises a first positioning sensor and a second positioning sensor. The first positioning sensor is disposed below a first electrode pair beginning at the head end of the annular distal end. The second positioning sensor is disposed below the electrode pair at a location of the mid-section of the annular distal end.

In the ablation catheter according to the first aspect of the invention, the annular diameter of the annular distal end is capable of shrinking.

In a natural state, the annular distal end may assume a helical shape, and the annular diameter may be 20-35 mm. The separation between the head end and the tail end of the annular distal end is 1/5~1/4 of the circumference of the annular ring.

The annular diameter may be 12~15 mm when the annular distal end is retracted to a minimum. The annular distal end is a closed ring in an annular cross-section, and the electrode pairs on the head end and the tail end of the annular distal end do not overlap.

In the ablation catheter according to the first aspect of the present invention, one end of the distal catheter body is a free end at the head end of the annular distal end. The free end has an atraumatic head end thereon. The other end of the tail end catheter body body is a fixed end. The catheter may further comprise: a tail end hard tube, one end of which is connected to a fixed end of the tail end catheter body; a distal catheter body, one end of which is connected to the other end of the tail end hard tube; a proximal catheter body, one end of which is connected to the other end of the distal catheter body; a handle assembly having one end connected to the other end of the proximal catheter body; and a connector connected to the other end of the handle assembly.

Preferably, the catheter may further comprise a support member penetratingly arranged inside the tail end catheter body and the tail end hard tube and being made of a memory alloy material; and a contraction rope disposed inside the loop of the support member, one end of which is fixed together with one end of the support member to the atraumatic head end. When the contraction rope contracts, the supporting member is deformed so as to adjust the diameter of the annular distal end.

Preferably, the catheter may further comprise a protective tube wrapped outside the support member and the contraction rope.

Preferably, the handle assembly has a knob thereon. The knob is configured to control retraction and recovery of the contraction rope by rotation.

Preferably, a positioning sensor is fixed on the outer circumference of the protective tube; the positioning sensor is of a cylindrical structure; and the protective tube passes through the center of the cylindrical structure.

Preferably, the positioning sensor is fixed to the protective tube with a sheath tube.

Preferably, the ablation catheter according to the first aspect of the present invention may further comprise a push button disposed between the handle assembly and the proximal catheter body. The push button is connected to one end of a traction assembly arranged in the distal catheter body, and the other end of the traction assembly is connected to the tail end hard tube, so that the push button is pushed to control the tightness of the traction assembly so as to achieve the bending of the distal catheter body.

Preferably, the traction assembly is arranged sideways in the distal catheter body.

In the ablation catheter according to the first aspect of the present invention, a positioning electrode may be provided on the tail end hard tube, and a third positioning sensor may be provided inside the tail end hard tube.

In the ablation catheter according to the first aspect of the invention, the connector comprises a first connector, connected to the positioning sensor by a first cable, configured for transmitting positioning information; and a second connector, respectively connected to the ring electrode on each electrode pair by a second cable, configured for transmitting ablation energy.

According to a second aspect of the invention, it provides a method for controlling the function of an electrode pair on an ablation catheter according to the first aspect of the invention. The ablation catheter being a pulsed electric field ablation catheter for ablation of the heart and its surrounding tissues The process comprises: in an ablation mode of the catheter, transmitting electric field energy with the same polarity to the two electrodes in each electrode pair, so that the two electrodes in the electrode pair are equivalent to the same electrode to apply a voltage to the corresponding tissue and transmit electric field energy with opposite polarity to the adjacent electrode pair, so as to realize the pulsed electric field ablation function; and in a mapping mode of catheter, the electrophysiological signals are separately collected by two electrodes in each electrode pair, so as to realize the electrophysiological mapping function.

According to a third aspect of the present invention, there is provided a computer readable medium having stored thereon instructions executable by a processor which, when executed by the processor, cause the processor to perform a method for controlling the function of the electrode pair on the ablation catheter according to the second aspect of the present invention.

As mentioned above, the current fixed-diameter circular pulse ablation catheter cannot achieve good abutment against the channel structure. The diameter is too large to enter the channel structure. The diameter is too small to achieve good abutment for the channel structure, thus affecting the efficiency and effect of ablation. At the same time, the pulsed electric field ablation needs a large single electrode area (the electrode length or width dimension is as large as possible) due to the need to avoid the safety risk caused by too concentrated electric field, but the circular catheter structure needs to be adjustable in diameter, while the large electrode area and the larger electrode length or width dimension are not applicable to the circular diameter adjustment, because the variable diameter needs to make compression change on the shape of circular segment. Too large electrode size will increase the length of local rigid segment of circular catheter, making it difficult to deform and shrink the circular catheter.

In order to ensure the safety of pulsed electric field ablation, the electrode spacing used for pulsed ablation is generally designed to be larger. However, too large electrode spacing can easily introduce other interference signals when collecting electrophysiological signals, and can not accurately collect local electrical signals, which can easily affect operator's judgment and surgical efficiency. Smaller electrodes and smaller electrode spacing are more desirable for mapping electrophysiological signals to achieve accurate mapping.

In view of this, the present invention proposes the use of electrode pairs. Thus, when used as an ablation electrode, a electrode pair may function equivalently as one electrode. When used as mapping electrodes, the two electrodes of the electrode pair are used separately. According to the design of the electrode pairs, on the premise of ensuring the ablation effectiveness and safety, the diameter of the annular distal end is more convenient to adjust. Moreover, the electrode pairs are used for collecting electrophysiological signals more accurately, and the situation that the change determination of electrical signals is affected by the large distance between the electrodes is avoided.

The positioning sensor helps to position and display the shape of the annular catheter. Due to the large compression deformation of the adjustable diameter ring segment, the conventional positioning sensor is rigid and difficult to deform with the deformation of the ring segment. However, if there is no positioning sensor, the annular pulse catheter has a very high safety risk. If the electrodes with different polarities are overlapped, there will be ignition problem, which will burn the heart tissue.

Thus, in a preferred embodiment of the invention, a freely deformable and performance-free positioning sensor arrangement is proposed for use on an adjustable diameter annular section of a catheter.

**Brief Description of the Drawings**

The invention will be more fully understood from the following detailed description taken in conjunction with the accompanying drawings, in which like elements are numbered in like fashion, and in which:

Fig. 1 is an overall schematic view of an ablation catheter according to the present invention.
Fig. 2 is a schematic view of the natural state of the annular distal end.
Fig. 3 is a schematic view of the natural electrode pair arrangement of the annular distal end.
Fig. 4 is a schematic view of the arrangement of the traction assembly of the distal catheter body.
Fig. 5 is a schematic view of a contracted state of the annular distal end.
Fig. 6 is a schematic view of the electrode pair arrangement in the collapsed state of the annular distal end.
Fig. 7 is a schematic view of a support member.
Fig. 8 is a schematic view of the interior of the annular distal end.
Fig. 9 is a schematic view of a positioning sensor arrangement in the annular distal end.
Fig. 10 is a side schematic view of the annular distal extension.

Fig. 11 is a cross-sectional view of the annular distal head end.

Fig. 12 is a cross-sectional view of the distal catheter body.

Fig. 13 is a schematic view of the ring electrode being a spiral electrode.

Fig. 14 is a schematic view of the bending effect of the distal catheter body.

Fig. 15 is an internal schematic view of the retraction control assembly of the annular distal end.

Fig. 16 is a application schematic view according to an embodiment of the present invention.

Fig. 17 is another application schematic view according to an embodiment of the present invention.

Fig. 18 is a schematic view of an electric field distribution when an ablation catheter according to the present invention releases electric field energy.

Reference numerals

1-Circular distal end
2-electrode pair
3-tail end catheter body
4-tail end rigid tube
41-positioning electrode
42-traction assembly
5-distal catheter body
6-proximal catheter body
7-support member
71-support member head end
72-support member proximal end
8-atraumatic head end
9-contraction rope
10-protective tube
11-ring electrode
12-ring electrode
131-first positioning sensor
132-second positioning sensor
133-third positioning sensor
14-sheath tube
15-handle assembly
16-push button
17-knob
171-sliding block
172-Rotating mandrel
173-sliding groove
18-first connector
19-second connector
20-cavity channel structure
111-first electrode pair
112-second electrode pair
113-third electrode pair
114-fourth electrode pair
115-fifth electrode pair
116-sixth electrode pair
117-seventh electrode pair
118-eighth electrode pair
119-ninth electrode pair
21-myocardial tissue
22-electric field region formed by discharge

**Detailed Description**

The embodiments of the present invention will be described in further detail with reference to the accompanying drawings, but the present invention is not limited to the following embodiments.

Ablation catheter

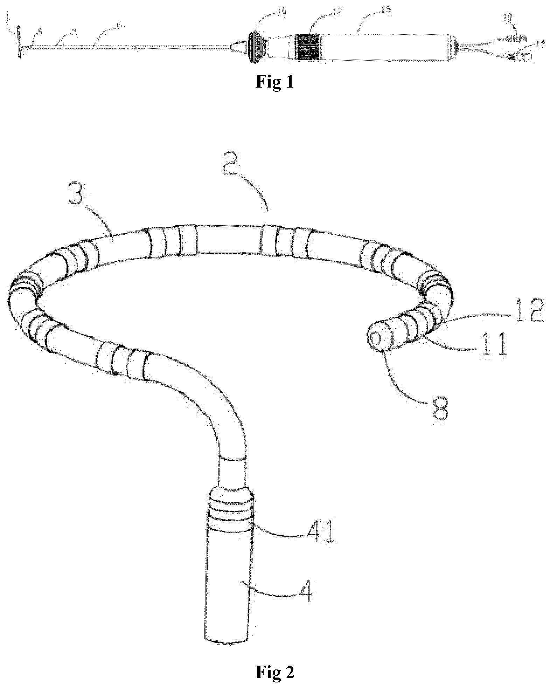

Fig. 1 is an overall schematic view of an ablation catheter according to the present invention.

As shown in Fig. 1, the ablation catheter according to the present invention may comprise an annular distal end 1, a tail end hard tube 4, a distal catheter body 5, a proximal catheter body 6, a handle assembly 15, and connectors 18 and 19 in the overall structure from the distal end to the proximal end. The annular distal end 1 is disposed at the distal-most end of the catheter. The tail end rigid tube 4 is used to connect the annular distal end 1 to the distal catheter body 5. The tail end hard tube 4 is preferably made of polyetheretherketone high molecular material with high hardness. The distal catheter body 5 is disposed between the proximal catheter body 6 and the tail end hard tube 4. The distal catheter body 5 is bendable by manipulation of the handle assembly 15 to bring the annular distal end 1 to the desired location. The proximal catheter body 6 is used to connect the distal catheter body 5 to the handle assembly 15. The connectors of the ablation catheter shown in Fig. 1 include at least two types of connectors: a connector 18 is used to transfer positioning information between the catheter and the device, and a connector 19 is used to transfer ablation energy to and from the device.

The ablation catheter shown in Fig. 1 also includes a push button 16 between the proximal catheter body 6 and the handle assembly 15, and a knob 17 on the handle assembly 15.

In the present disclosure, "distal" and "proximal" are relative to the catheter operator, e.g., the distal end may be the end closer to the tissue to be ablated and the proximal end may be the end closer to the catheter operator. In addition, the "head end" and the "tail end" generally refer to a free end and a fixed end (or "connecting end ").

It will be appreciated by those skilled in the art that while the above-described elements and components are combined to form one preferred embodiment of an ablation catheter according to the present invention, the ablation catheter according to the present invention is not limited to including all such elements and components.

Fig. 2 is a schematic view of the natural state of the annular distal end. As shown in Fig. 2, the annular distal end has an outer diameter of 20-35 mm in its natural state, presenting the appearance of the letter "C". The head end of the annular distal end is an atraumatic head end 8 and the tail end is a hard end. The head end is separated from the tail end by a distance of 1/4 to 1/5 of the generally circular configuration. The annular distal end naturally has a spiral shape, and the annular distal end is provided with a tail end catheter body 3 having an arc shape corresponding to the shape of the annular distal end. The tail end catheter body 3 is preferably supported by a highly elastic polyurethane material. The tail end catheter body 3 is provided with a plurality of electrode pairs 2. In a preferred embodiment of the invention, the number of electrode pairs is 2N+1, N being a positive integer. Each electrode pair consists of two electrodes. For example, the first electrode pair closest to the head end (atraumatic head end 8) is composed of a ring electrode 11 and a ring electrode 12.

Briefly, the ablation catheter according to the present invention has an annular distal end 1. The annular distal end 1 comprises an arc-shaped tail end catheter body 3. The innovation of the present invention is that a plurality of electrode pairs are provided at intervals along the arc-shaped extending direction of the tail end catheter body. The plurality of electrode pairs 2 are respectively wound around the tail end catheter body 3.

Fig. 2 also shows the tail end hard tube 4 and the positioning electrode 41 provided thereon, as will be described in more detail below.

With reference to Figs. 1 and 2, it can be seen that one end of the tail end catheter body 3 is a free end and is at the head end of the annular distal end 1. The free end has an atraumatic head end 8. The other end of the tail end catheter body 3 is a fixed end. One end of the tail end hard tube 4 is connected to the fixed end of the tail end catheter body 3. One end of the distal catheter body 5 is connected to the other end of the tail end hard tube 4. One end of the proximal catheter body 6 is connected to the other end of the distal catheter body 5. One end of the handle assembly 15 is connected to the other end of the proximal catheter body 6. A push button 16 may be provided between the handle assembly 15 and the proximal catheter body 6. In addition, a knob 17 may be provided on the handle assembly 15. Connectors 18, 19 are connected to the other end of handle assembly 15.

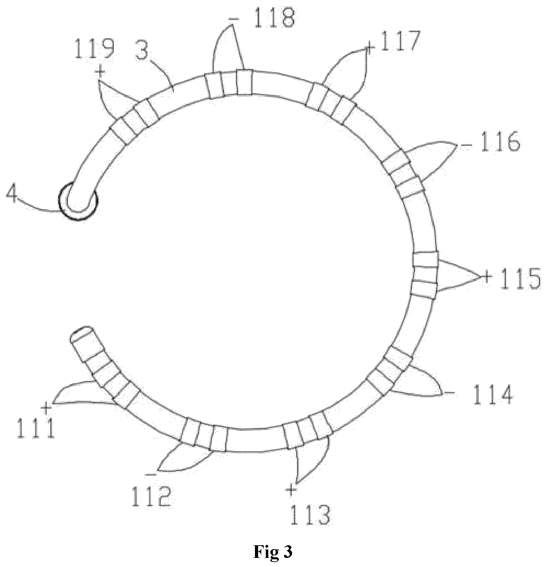

Fig. 3 is a schematic view of the natural electrode pair arrangement of the annular distal end. For example, as shown in Fig. 3, the first electrode pair 111 is composed of two minute ring electrodes. The ring electrode is preferably a gold or platinum electrode having a dimension (which may be referred to as "length" or "width ") in the direction of the arc-shaped extent of the tail end catheter body of from 0.50 mm to 1.5 mm. The clearance gap between ring electrodes is 1-3 mm. The clearance spacing between the ring electrode pairs is 3-6 mm. It should be noted here that the design of the electrode width, spacing, etc. used here is such that, since the diameter of the annular distal end can be contracted, the electrode size needs to be adapted thereto, in particular the electrode size in the arc-shaped extending direction of the tail end catheter body, i.e., the so-called "length" or "width" width ", needs to be appropriate, and an excessive size will affect the contraction effect of the annular distal end. Thus, it is provided that two annular electrodes constitute one electrode pair.

In short, each electrode pair 2 comprises two ring electrodes, for example ring electrodes 11 and 12, encircling the tail end catheter body 3. The width of each ring electrode in the direction of the arc-shaped extension of the tail end catheter body, the spacing of the two ring electrodes in the same electrode pair, the spacing between adjacent electrode pairs needing to be set to a suitable size. In a preferred embodiment, there is a constraint between these dimensions and the diameter of each ring electrode (described in more detail below).

The terms "dimension in the direction of arc-shaped extension of the distal catheter body", "spacing" and the like are used in this disclosure to refer to the dimension over the annular or arc-shaped perimeter of the annular distal end in contact with the myocardial tissue to be ablated, and not to refer to the dimension in the thickness of the distal catheter body or the annular distal end. It will be understood by those skilled in the art that such dimensions may be described as either "length" or "width", and by default, either "length" or "width" refers to a dimension on the circumference of the annulus and does not refer to a dimension in the thickness of the tube.

As previously mentioned, 2N+1 (odd) ring electrode pairs may be provided on the annular distal end. As shown in Fig. 3, during the discharge ablation, the first electrode pair to the ninth electrodes 111-119 are alternately loaded with positive and negative voltages. For example, the first electrode pair 111 is loaded with a positive electrode and the second electrode pair 112 is loaded with a negative electrode, which are sequentially loaded in sequence, that is to say, the polarities of adjacent electrode pairs are opposite. The last electrode pair in Fig. 3 is the ninth electrode pair 119, which has a positive polarity. Since the number of electrode pairs is an odd number, the polarity of the last electrode pair is the same as that of the first electrode pair. For example, the polarity of both the first electrode pair 111 and the ninth electrode pair 119 is positive. The purpose of this arrangement is to ensure that when the distal end of the the electrodes overlap when the annular distal end is retracted, thereby ensuring safety in use. In the ablation mode, the two electrodes in the electrode pair have the same polarity during discharge ablation, which can be equivalently regarded as one electrode. The split electrode pair design (consisting of two ring electrodes) reduces the stiffness of the distal catheter body and facilitates dimensional contraction of the annular distal end.

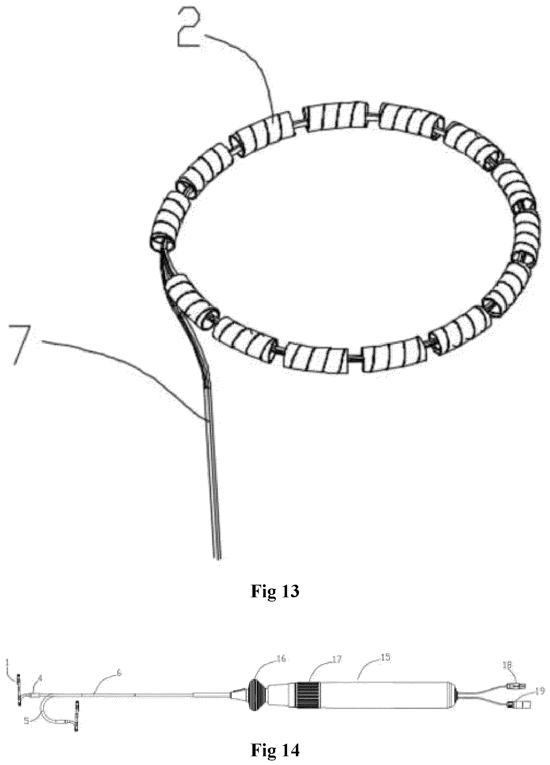

As shown in Fig. 13, the ring electrode may be a spiral electrode. The spiral electrode is an electrode in which a lead is wound or a ring electrode is cut into a spiral shape.

Referring back to Fig. 3, when the ring electrode is placed on the distal catheter body 3, the rigidity of the distal catheter body can be reduced, facilitating the contraction of the annular distal diameter. The electrode pairs 111-119 are each ring-shaped, preferably in equally spaced fashion, around the tail end catheter body 3. The tail end catheter body 3 is connected to an tail end hard tube 4. The electrode material may be platinum iridium alloy, gold.

In a preferred embodiment of the invention, the ablation catheter is a pulsed electric field ablation catheter for delivering and releasing pulsed ablation energy to a desired ablation site. That is, the ablation catheter is mainly used to perform ablation by applying a high voltage pulsed electric field to human tissues. More specifically, the ablation catheter according to the present invention may be used for ablation of the heart and its surrounding tissue.

Since the pulsed electric field ablation applies high voltage between electrodes to perform discharge ablation, with voltage of 1000-4000V, it is required that the electrode surface area shall be large enough. Otherwise, the local field intensity shall be too concentrated to generate arc phenomenon during discharge. However, as previously mentioned, the problem of annular distal diameter adjustment is taken into account, so that the electrodes are arranged in electrode pair arrangements. A single electrode is separated by a short distance and then provided with an electrode, so that the two electrodes can form an electrode pair. When discharging, the electrode pair (electrode pair) is equivalently regarded as one electrode, and the discharging effect of the electrode with a sufficiently large surface area is exerted without affecting the diameter adjustment of the annular distal end because the size of the electrode is too large.

On the other hand, the two electrodes in the electrode pair are closely spaced and can be used separately for mapping at the same time. Since the energy output of pulsed electric field is high and concentrated, it is required that the distance between the loaded positive and negative electrodes shall be as large as possible. However, too large electrode spacing will make the acquired electrophysiological signal range too large, affecting the judgment on local ablation effect. Therefore, after the ablation is completed, two closely spaced electrodes in the electrode pair are used to collect the electrophysiological signal, thereby judging the electrophysiological signal more accurately, and local electrical signal can be judged more finely, so as to avoid introducing a large range of electrophysiological signal due to the excessive electrode spacing and affecting the judgement of ablation effect.

In short, therefore, when used for pulsed electric field ablation, the ablation catheter is in ablation mode and the two electrodes of each electrode pair are of the same polarity, thereby being equivalent to the same electrode to which the voltage is applied, thereby acting as a discharge of an electrode of sufficiently large surface area upon ablation. At the same time, the polarity of adjacent pairs of electrodes is opposite.

On the other hand, when used for electrophysiological mapping, the ablation catheter is in mapping mode, and the two electrodes in each electrode pair respectively function, i.e., are used to collect electrophysiological signals separately, and function as two electrodes with relatively close distance.

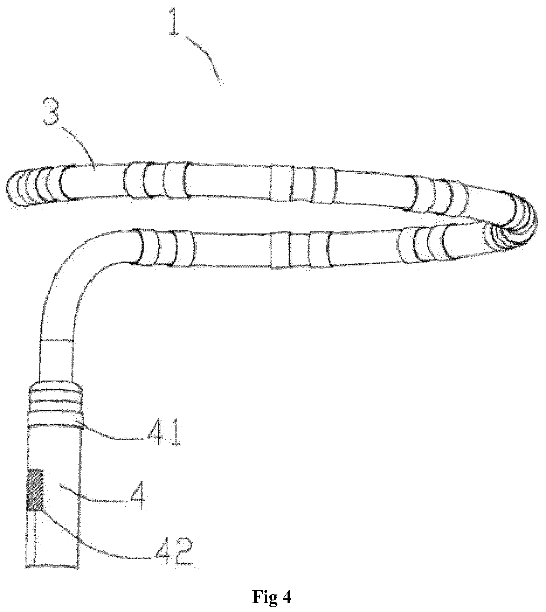

Fig. 4 is a schematic view of the arrangement of the traction assembly of the distal catheter body. As shown in Fig. 4, the annular distal end 1 is disposed on a tail end rigid tube 4. An unbent section of the distal catheter body 3 in the proximal direction is connected to the tail end rigid tube 4. A traction assembly 42 is disposed within the tail end hard tube 4. A traction assembly 42 is secured to the tail end hard tube 4 to effect bending of the distal catheter body 5 under the control of a push button 16 between the handle assembly 15 and the proximal catheter body 6 (see Fig. 14). A positioning electrode 41 is provided on the tail end hard tube 4 for cooperating with the positioning of the positioning sensor, such as the positioning sensors 131, 132, 133 described below.

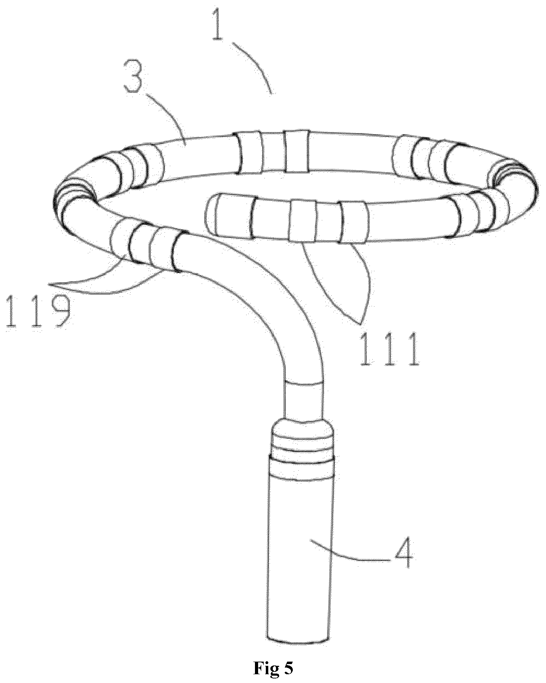

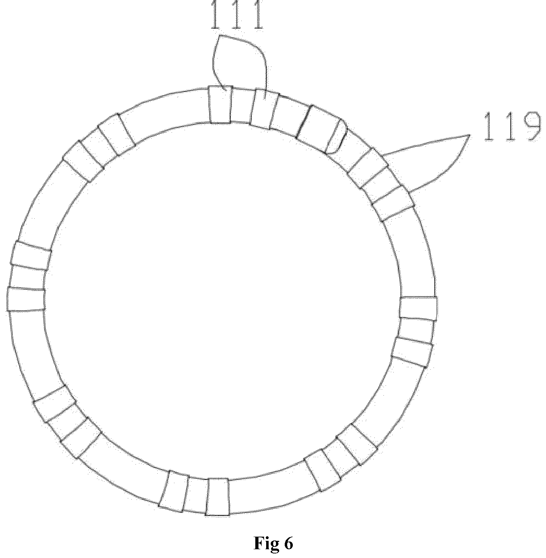

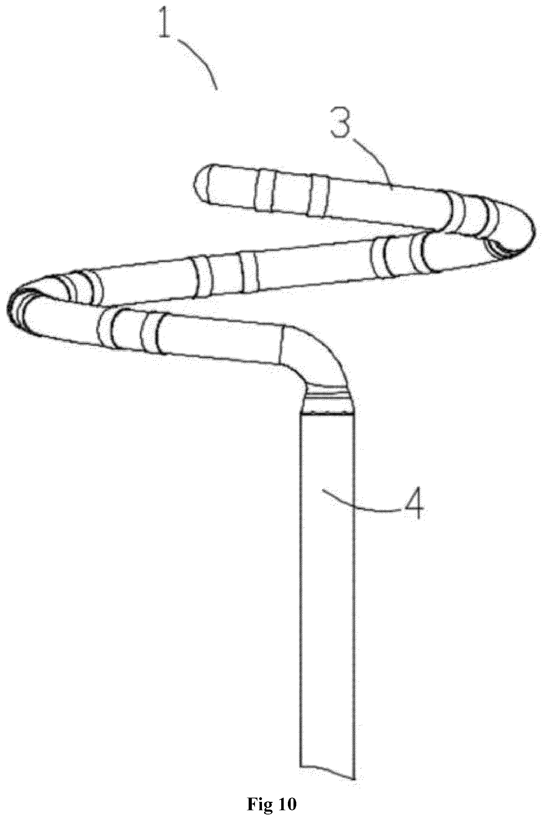

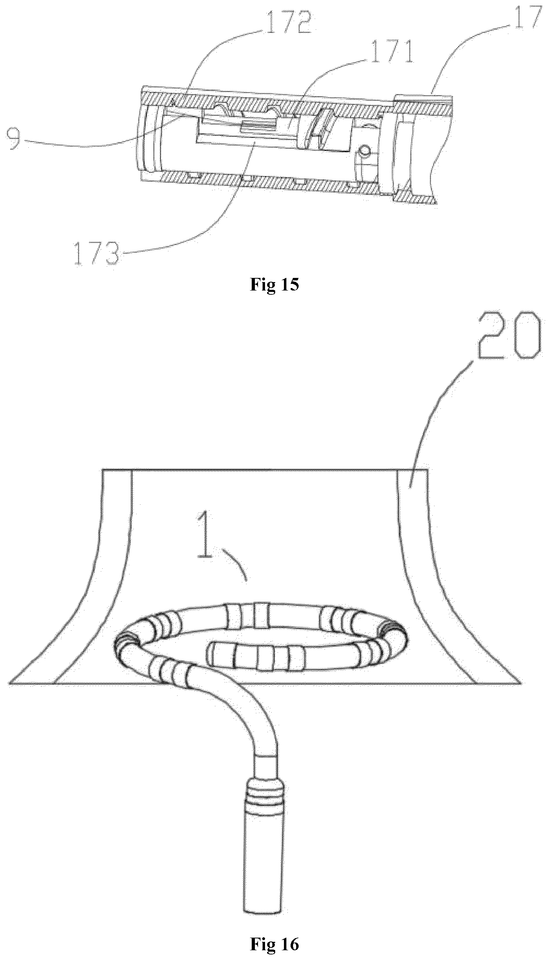

Fig. 5 is a schematic view of a contracted state of the annular distal end. Fig. 6 is a schematic view of the electrode pair arrangement in the collapsed state of the annular distal end. Fig. 10 is a side schematic view of the annular distal extension. Fig. 15 is an internal schematic view of the retraction control assembly of the annular distal end.

As previously mentioned, the handle assembly 15 may have a knob 17 thereon. By rotating the knob 17, the retraction and recovery of the contraction rope can be controlled to effect a change in the annular diameter of the annular distal end.

Referring to Figs. 5, 6, 10 and 15, and more particularly to Fig. 15, when the knob 17 on the handle assembly 15 is manipulated, the sliding block 171 moves the contraction rope 9. The contraction rope 9 and the sliding block 171 are fixed together. The rotating mandrel 172 is internally provided with an internal thread to mate with the external thread on the sliding block 171. When the knob 17 is rotated clockwise, the knob 17 will rotate the rotating mandrel 172, which will cause the sliding block 171 to move backwards, pulling the contraction rope 9, thereby causing the annular distal end 1 to retract. When the knob 17 is rotated counterclockwise, the contraction rope 9 recovers and the diameter of the annular distal end 1 is increased or recovered.

As previously mentioned, in the natural state, the annular distal end assumes a helical annular shape with an annular diameter of 20-35 mm. The separation between the head end and end of the annular distal end 1 is 1/5-1/4 of the circumference of the annular ring. As shown in Figs. 5, 6 and 10, when the annular distal end 1 is contracted to a minimum, the two end-to-end electrode pairs 111 and 119 on the distal tube 3 do not overlap, further increasing safety. When the diameter of the distal end of ring is reduced to the minimum, the distal end of ring 1 presents a closed circular ring on the ring section, with the diameter of ring being 12-15 mm. The electrode pairs on the head end and the tail end are not overlapped. In addition to the tail end catheter body 3 and electrode pairs (e.g. 111 and 119) shown in Figs. 5 and 10, the tail end hard tube 4 or a portion thereof is shown.

The ablation catheter according to the present invention may further include a support member penetratingly disposed inside the distal catheter body and the tail end hard tube, and a contraction rope disposed at an annular inner side of the support member. One end of the contraction rope is secured to the atraumatic head end together with one end of the support member. As the contraction rope retracts, the support member deforms to adjust the annular diameter of the annular distal end.

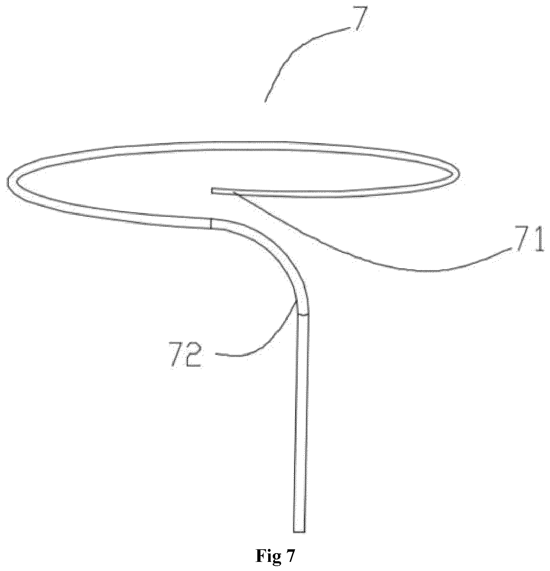

Fig. 7 is a schematic view of a support member. As shown in Fig. 7, the support member 7 has a ring shape. The shape of the annular distal end is primarily determined by the shape of the support member. The material of the supporting member is a memory alloy material with high elasticity, such as NiTi, which can instantly recover the original shape after removing the external force. In a preferred embodiment, the support member head end 71 is not of the same diameter as the support member proximal end 72. The diameter of the support member head end 71 is smaller than the diameter of the support member proximal end 72, and the diameter increases gradually from the head end to the proximal end in order to achieve the adjustable diameter function of the annular distal end. In order to achieve the contraction of the ring-shaped distal end 1, the ring-shaped distal end 1 contracts under the action of the contraction rope 9. However, if the rigidity of the head end is too great, the deformation and contraction of the head end cannot be achieved. Therefore, the diameter of the head end is smaller than that of the proximal end and gradually increases, with the diameter of the head end being 1/3-4/5 of that of the proximal end. The contraction rope 9 is preferably a flexible and high tensile strength stainless steel wire rope with a diameter of 0.10-0.15 mm. The soft contraction rope 9 will not cause the rigidity of the annular distal end 1 to increase, thereby affecting the deformation of the annular ring. The contraction rope 9 may also be a polyethylene rope (PE rope), be soft and have a high tensile strength. The contraction ropes 9 are described in more detail below.

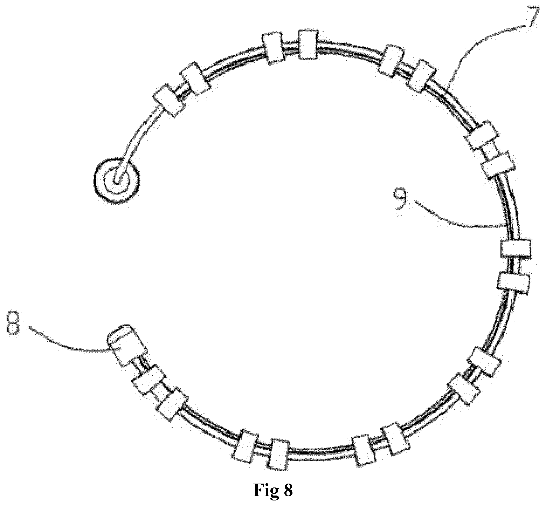

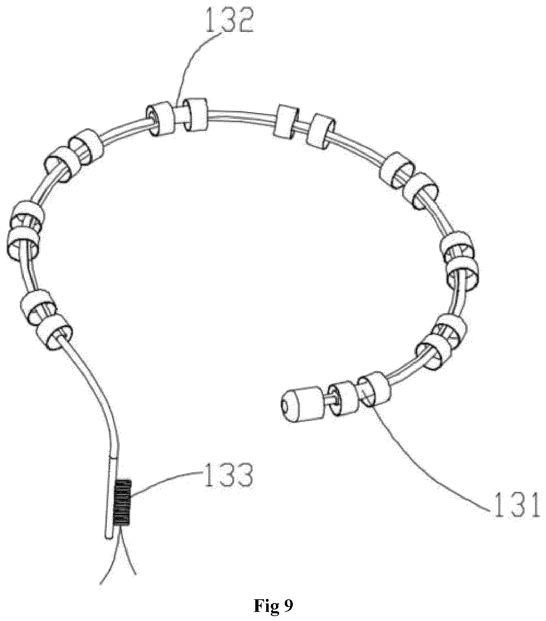

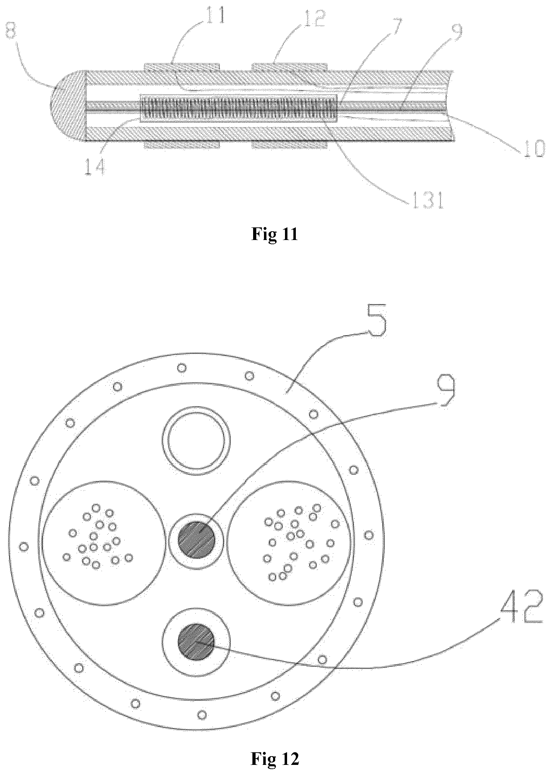

Fig. 8 is a schematic view of the interior of the annular distal end. Fig. 9 is a schematic view of a positioning sensor arrangement in the annular distal end. Fig. 11 is a cross-sectional view of the annular distal head end.

As shown in Fig. 8, the contraction rope 9 is provided inside the loop of the support member 7 to deform the support member 7 by the contraction rope 9, thereby causing a change in the diameter of the loop-shaped distal end 1, i.e., allowing the loop-shaped diameter of the loop-shaped distal end 1 to contract. The support member 7 is secured together with the contraction rope 9 inside the atraumatic head end 8 at the head end of the annular distal end 1. As shown in Fig. 11, the protective tube 10 is used to wrap around the outside of the support member 7 and the contraction rope 9. The protective tube 10 serves to protect the contraction rope 9 from affecting devices inside the tail end catheter body 3 when contracting, while making the contraction of the contraction rope 9 smoother. The material of the protective tube 10 is preferably Teflon tubing.

In a preferred embodiment of the present invention, a positioning sensor may be disposed below the electrode pair such that the positioning sensor is inside the distal tube.

As shown in Fig. 9, the first positioning sensor 131 is disposed at the head end of the annular distal end 1, below the first electrode pair 111. The second positioning sensor 132 is disposed at a location intermediate the annular distal end 1, such as may be below the electrode pair at a location intermediate the annular distal end 1, such as below the sixth electrode pair 116. The third positioning sensor 133 is arranged inside the tail end hard tube 4. These positioning sensors, in combination with the positioning electrodes 41 (Figs. 2 and 4) and the electrode on the distal tube 3, can display the shape of the annular distal end 1 and the positional relationship of the electrodes, so as to facilitate the observation of whether there is overlap between the electrodes, so as to better ensure the safety of the electrodes. The position of the easiest overlap may be monitored by means of a magnetic positioning function to limit the energy output in extreme situations. The positioning sensor in the direction of the arc-shaped extension of the tail end catheter body has a dimension, which may for example be referred to as "length", of being equal to the sum of the respective dimensions of the two ring electrodes ("length" or "width" in the direction of the arc-shaped extension of the tail end catheter body) and their spacing, i.e., the dimension of one electrode pair (width of one electrode pair in the direction of the arc-shaped extension of the tail end catheter body), and the positioning sensor is arranged directly below the electrode pair. This is designed to avoid increasing the rigidity of the annular section. The positioning sensor has a cylindrical structure with a hollow center. Thereby, the support member 7 may pass through the center of the cylindrical structure and fix the positioning sensor on the support member 7. The positioning sensor is a cylindrical structure spirally wound by the copper wire, which has certain elasticity. After being protected and fixed by the polyurethane material with high elasticity on the outside, it forms a positioning sensor which may be freely deformed and has no effect on performance, and is suitable for use on annular adjustable annular ring.

As shown in Fig. 11, each positioning sensor is fixed to the support member 7. In order to be stably fixed thereon, the first positioning sensor 131 and the protective tube 10 (including the support member 7 inside the protective tube 10, the contraction rope 9) are fixed with the sheath tube 14. Similarly, the second positioning sensor 132 may be secured to the protective tube 10 with the sheath tube 14. The material of the sheath tube 14 is preferably a highly elastic polyurethane pipe having high elasticity and facilitating adhesion. The support member 7, the contraction rope 9 and the protective tube 10 are secured together at the atraumatic head end 8. In the illustration of Fig. 11, the positioning sensor 131 is disposed at the head end of the annular distal end 1, beneath the first pair of ring electrodes 11 and 12. The dimension (e.g., may be referred to as "length ") of the positioning sensor 131 in the arc-shaped extending direction of the end pipe is equal to the dimension ("length" or "width" in the arc-shaped extending direction of the end pipe) of the two ring electrodes 11 and 12 and the spacing thereof, i.e., the dimension of one electrode pair, and the positioning sensor 131 is disposed directly below the pair of electrodes 11 and 12.

As shown in Fig. 11, the first positioning sensor 131 is fixed on the outer circumference of the protective tube 10. Since the first positioning sensor 131 has a cylindrical structure with a hollow center, the protective tube 10 may pass through the center of the cylindrical structure of the first positioning sensor 131. That is, the support member 7 passes through the center of the cylindrical structure of the first positioning sensor 131.

Similarly, the second positioning sensor 132 may be fixed on the outer circumference of the protective tube 10. Since the second positioning sensor 132 has a cylindrical structure with a hollow center, the protective tube 10 can pass through the center of the cylindrical structure of the second positioning sensor 132. That is, the support member 7 passes through the center of the cylindrical structure of the second positioning sensor 132.

Fig. 12 is a cross-sectional view of the distal catheter body. Fig. 14 is a schematic view of the bending effect of the distal catheter body.

As previously described, the ablation catheter according to the present invention may further include a push button 16 disposed between the handle assembly 15 and the proximal catheter body 6. In accordance with a preferred embodiment of the present invention, the push button 15 is connected to one end of a traction assembly 42 disposed within the distal catheter body 5. The other end of the traction assembly 42 is connected to the tail end hard tube 4. By pushing the push knob 16, the tightness of the traction assembly 42 can be controlled, whereby the bending of the distal catheter body 5 is achieved.

As shown in Figs. 12 and 14, pushing the push button 16 controls the bending of the distal catheter body 5 to help the annular distal end 1 reach the target position. As shown in Fig. 12, the distal catheter body 5 is a multi-lumen tube structure, and the contraction rope 9 of the annular distal end 1 is placed in the middle lumen. Because of its placement in the middle lumen, the contraction rope 9 does not interfere with the bending of the distal catheter body 5 as it retracts and causes the distal catheter body 5 to bend. The traction assembly 42 is provided on the side of the distal tube 5 to facilitate bending of the distal tube 5 without affecting the intermediate contraction ropes 9. The contraction rope 9 is in the middle and does not cause relative movement of the contraction rope 9 whether the distal catheter body 5 is in a bent or straightened state. This avoids the problem of contraction of the annular distal end 1 and possible interference with the bending of the distal catheter body 5.

Fig. 14 shows the same elements and components as in Fig. 1, i.e., including, in addition to the push button 16 and the knob 17, the annular distal end 1, the distal catheter body 5, the proximal catheter body 6, the handle assembly 15, and the connectors 18 and 19. It will be appreciated by those skilled in the art that while the above-described elements and components are combined to form one preferred embodiment of an ablation catheter according to the present invention, the ablation catheter according to the present invention is not limited to including all such elements and components.

In addition, as previously described, The connectors of the ablation catheter shown in Figs. 1 and 14 include at least two types of connectors: a connector 18 is used to transfer positioning information between the catheter and the device, and a connector 19 is used to transfer ablation energy to and from the device. Referring to the depiction of Fig. 11, the connector 18 is connected to the positioning sensor by a first cable to transmit positioning information. The connector 19 is respectively connected to the ring electrode on each electrode pair via a second cable so as to transmit ablation energy. The connector 19 and the second cable may also be used to acquire electrophysiological mapping signals when the ablation catheter is in the mapping mode.

Specific application example

The specific use of the annular variable distal diameter ablation catheter according to the present invention will now be described in more detail by way of application examples.

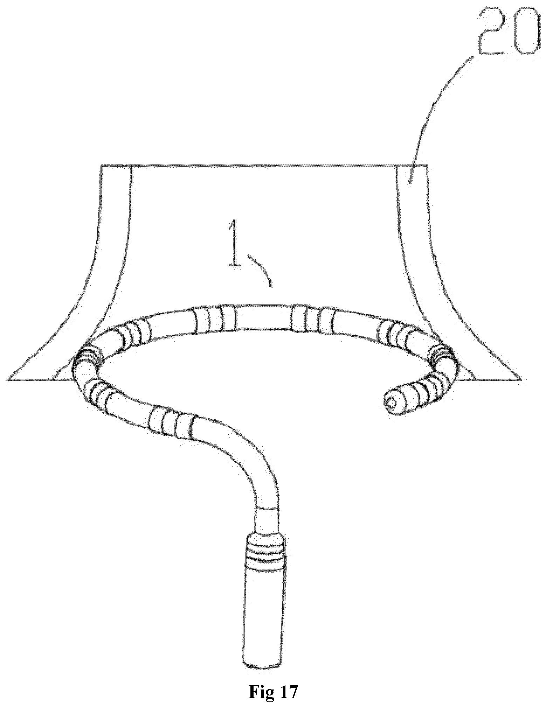

Fig. 16 is an application schematic view according to an embodiment of the present invention. Fig. 17 is another application schematic view according to an embodiment of the present invention.

In the application embodiment of Figs. 16 and 17, the following operational steps may be included.

Step 1, as shown in Fig. 16, the diameter of the annular distal end 1 is shrunk to a small diameter annular distal end 1, and then extends into the cavity channel structure 20.

Step 2: the electrophysiological signal mapping is performed inside the cavity structure 20 and a physical model of the cavity structure 20 is constructed.

Step 3: as shown in Fig. 17, control to increase the diameter of the ring-shaped distal end 1, so that the electrode can make good contact with the cavity channel structure 20.

Step 4: it is discharged for the ablation, with the voltage amplitude of 1000-4000V After ablation, the electrophysiological signals are collected by the two electrodes in the electrode pair. The immediate ablation effect may be determined by comparing changes in the electrophysiological signal until the electrical activity of the tunnel structure 20 is completely isolated.

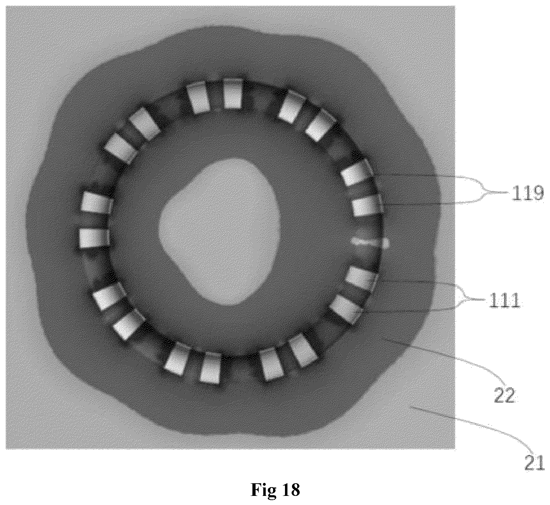

Fig. 18 is a schematic view of an electric field distribution when an ablation catheter according to the present invention releases electric field energy.

As shown in Fig. 18, when a pulsed discharge is applied, all electrodes on the ablation catheter form a continuous ablation zone in the myocardial tissue 21, i.e., the electric field region 22 formed by the discharge shown in the drawing. Typically, the myocardial ablation effectiveness threshold is 400V/CM. In the electric field region 22, the voltage intensity is greater than 400V/CM, reaching this threshold, so that effective myocardial ablation can be performed over this continuous region. The number of electrode pairs is 2N+1 (N is a positive integer). Each electrode pair consists of two ring electrodes, as shown in Fig. 18, the number of electrode pairs is nine. Either the first electrode pair 111 or the ninth electrode pair 119 is composed of two minute ring electrodes. The ring electrodes are preferably gold or platinum electrodes having a dimension ("length" or "width" in the direction of arc-shaped extension of the tail end catheter body) of from 0.50 to 1.5 mm. A clearance spacing between ring electrodes is from 1 to 3 mm, a clearance spacing between pairs of ring electrodes is from 3 to 6 mm, and an applied pulsed electric field has an amplitude of from 1000V to 4000V To improve the ablation efficiency, a continuous ablation band should be formed during electrode discharge to prevent ablation leakage points. According to the electric field distribution and mechanical design formed by electrode, under the premise of ensuring that the surface area of electrode pair is sufficiently large and the spacing between electrode pairs is appropriate, continuous ablation zone is formed during discharge ablation and annular adjustable function is ensured, which has the following relationship:

$$(2 \times L + d) \times k = D, \text{ and } S > D,$$

where L is the width (or referred to as "length") of the ring electrode in the arc-shaped extending direction of the tail end catheter body; d is the spacing between two ring electrodes in the same electrode pair; D is the spacing between adjacent electrode pairs; S is the diameter of each ring electrode; and k is a correction factor and has a value of 0.7-1.4 (k = 0.7-1.4). As previously mentioned, in a preferred embodiment, L has a value of 0.50-1.5 mm; d has a value of 1-3 mm; and D has a value of 3-6 mm.

It will be understood by those skilled in the art that, although various elements and components of the ablation catheter have been referred to in the various embodiments above, these elements and components may be combined in any combination within the ablation catheter to perform their respective functions, unless specifically excluded from the above description or otherwise not permitted or achievable in practice. None of the above examples or embodiments limit the scope of the present invention.

Electrode function control method

Since the ablation catheter of the present invention can be used in an ablation mode and a mapping mode, respectively, the electrodes and electrode pairs on the ablation catheter can be controlled accordingly so as to perform different functions in different modes, respectively, to achieve different functions.

In particular, the present invention proposes a method for controlling the function of an electrode pair on an ablation catheter according to the present invention. The ablation catheter is specifically a pulsed electric field ablation catheter used for ablation of the heart and its surrounding tissues. According to the proposed method of the present invention, in the ablation mode of the catheter, the electric field energy of the same polarity is delivered to the two closely spaced electrodes in each electrode pair, so that the two electrodes in the electrode pair are equivalent to the same electrode with a larger size or surface area and the electric field energy of opposite polarity is delivered to the adjacent electrode pair, so as to realize the pulsed electric field ablation function. On the other hand, in the mapping mode of catheter, the two electrodes with smaller spacing in each electrode pair are used separately, i.e., the electrophysiological signals are collected separately by the two electrodes in each electrode pair, so as to realize the electrophysiological mapping function.

The computer program or computer program product and computer readable medium

Moreover, those of ordinary skill in the art will recognize that the methods of the present disclosure may be implemented as computer programs. The methods described above are performed by one or more programs, including instructions to cause a computer or processor to perform a corresponding algorithm. These programs may be stored using various types of non-transitory computer-readable media and provided to a computer or processor. The non-transitory computer readable media include various types of tangible storage media. Examples of non-transitory computer-readable media include magnetic recording media such as floppy disks, magnetic tape, and hard disk drives, magneto-optical recording media such as magneto-optical disks, CD-ROM, CD-R, CD-R/W, and semiconductor memories such as ROM, PROM, EPROM, flash ROM, and RAM. Further, the programs may be provided to the computer using various types of instant computer-readable media. Examples of transient computer readable media include electrical signals, optical signals, and electromagnetic waves. The instant computer-readable medium can be used to provide a program to a computer via a wired communication path or a wireless communication path, such as through electrical wiring and fiber optics.

For example, according to one embodiment of the present disclosure, a computer-readable medium may be provided having stored thereon instructions executable by a processor, which when executed by the processor, cause the processor to perform a method for controlling the function of an electrode pair on an ablation catheter as previously described.

According to the present disclosure, it is also possible to propose a computer program or a computer program product which, when executed, can carry out the method for controlling the function of an electrode pair on an ablation catheter as described above.

In addition, the invention also relates to a computing device or computing system comprising a processor and a memory in which a computer program is stored which, when being executed by the processor, carries out the method for controlling the function of an electrode pair on an ablation catheter as previously described.

Beneficial effects

In summary, the ablation catheter according to the present invention has at least the following beneficial effects.

1. The design of pulse catheter with the adjustable annular distal end size is applicable to different cavity structures, and the adjustable mode facilitates that the electrode on annular distal end can better contact with the tissue structure.

2. According to the design of the electrode pairs, on the premise of ensuring the ablation effectiveness and safety, the diameter of the annular distal end is more convenient to adjust. Moreover, the electrode pairs are used for collecting electrophysiological signals more accurately, and the situation that the change determination of electrical signals is affected by the large distance between the electrodes is avoided.

3. The electrode design of this invention has that the electrode pair is merged into one electrode for ablation during ablation, and the electrode pair is separated into two electrodes for mapping during mapping, which fully takes into account the effectiveness of pulsed electric field ablation and the accuracy of electrophysiological mapping.

4. It is proposed that the positioning sensor which can be freely deformed without affecting the performance is applicable to the adjustable diameter annular segment of catheter, so that the ablation catheter can provide real-time display of morphology and electrode spacing in operation, so as to guide the operator in accurate ablation operation, and can monitor the electrode spacing to increase the safety.

5. The high-voltage pulse energy is accurately and effectively applied to the target tissue, greatly reducing the operation time. The high-voltage pulse energy can selectively ablate the target tissue, reducing complications.

6. The catheter has the functions of mapping, modeling and elimination, which can save operation time and cost.

The embodiments of the present invention are not limited to the embodiments described above, and various changes and modifications in form and detail may be made by those skilled in the art without departing from the spirit and scope of the present invention, which should be considered as falling within the scope of the present invention.

## Claims

1. An ablation catheter having an annular distal end (1) comprising an arc-shaped tail end catheter body (3), wherein a plurality of electrode pairs (2) are arranged at intervals along the arc-shaped extending direction of the tail end catheter body, and the plurality of electrode pairs (2) are respectively wound on the tail end catheter body (3).

2. The catheter according to claim 1, wherein the number of electrode pairs (2) is 2N+1, where N is a positive integer.

3. The catheter according to claim 1, wherein each electrode pair (2) comprises two ring electrodes (11, 12) surrounding the tail end catheter body (3); the width of each ring electrode in the arc-shaped extending direction of the tail end catheter body is L; the spacing between two ring electrodes in the same electrode pair is d; the spacing between adjacent electrode pairs is D; and the diameter of each ring electrode is S, then:

$$S>D=(2\times L+d)\times k,$$

where k is a correction factor and k ranges from 0.7 to 1.4.

4. The catheter according to claim 3, wherein L has a value of 0.50-1.5 mm; d has a value of 1-3 mm; and D has a value of 3-6 mm.

5. The catheter according to claim 3, wherein the ring electrode is a spiral electrode.

6. The catheter according to claim 5, wherein the helical electrode is wound from an electrode cable.

7. The catheter according to claim 5, wherein the spiral electrode is an electrode that cuts the ring electrode into a spiral form.

8. The catheter according to claim 1, wherein the catheter is a pulsed electric field ablation catheter for delivering and releasing pulsed ablation energy to a desired ablation site.

9. The catheter according to claim 8, wherein the catheter is used for ablation of the heart and surrounding tissues.

10. The catheter according to claim 8, wherein, when used for the pulsed electric field ablation, the two electrodes of each electrode pair are of the same polarity, equivalent to applying a voltage to the same electrode, and adjacent electrode pairs are of opposite polarity.

11. The catheter according to claim 8, wherein the two electrodes of each electrode pair are each independently configured for acquiring electrophysiological signals when used for electrophysiological mapping.

12. The catheter according to claim 10, wherein the voltage amplitude applied by each electrode pair is 1000-4000V

13. The catheter according to claim 1, wherein a positioning sensor (131, 132) is arranged below the electrode pair (2), inside the tail end catheter body (3).

14. The catheter according to claim 13, wherein the length of the positioning sensor (131, 132) in the arc-shaped extension of the tail end catheter body is equal to the width of the electrode pair (2) in the arc-shaped extension of the tail end catheter body.

15. The catheter according to claim 14, wherein the length of the positioning sensor (131, 132) in the direction of the arc-shaped extension of the tail end catheter body is equal to the sum of the width of the two ring electrodes of the electrode pair respectively in the direction of the arc-shaped extension of the tail end catheter body and their spacing.

16. The catheter according to claim 13, wherein the positioning sensor comprises a first positioning sensor (131) disposed below a first electrode pair beginning at a head end of the annular distal end and a second positioning sensor (132) disposed below the electrode pair at a location of the mid-section of the annular distal end.

17. The catheter according to claim 1, wherein the annular diameter of the annular distal end is capable of shrinking.

18. The catheter according to claim 17, wherein, in a natural state, the annular distal end has a helical shape with an annular diameter of 20-35 mm; and the separation between the head end and the tail end of the annular distal end is 1/5-1/4 of the circumference of the annular ring.

19. The catheter according to claim 18, wherein the annular diameter is 12-15 mm when the annular distal end is contracted to a minimum, wherein the annular distal end is a closed circular ring in an annular cross-section, and the electrode pairs on the head end and the tail end of the annular distal end do not overlap.

20. The catheter according to claim 1, wherein one end of the tail end catheter body (3) is a free end at the head end of the annular distal end (1), the free end having an atraumatic head end (8) thereon, and the other end of the tail end catheter body (3) is a fixed end;
the catheter further comprises:

a tail end hard tube (4), one end of which is connected to a fixed end of the tail end catheter body (3);
a distal catheter body (5), one end of which is connected to the other end of the tail end hard tube (4);
a proximal catheter body (6), one end of which is connected to the other end of the distal catheter body (5);
a handle assembly (15) having one end connected to the other end of the proximal catheter body (6); and
a connector (18, 19) connected to the other end of the handle assembly (15).

21. The catheter according to claim 20, wherein the catheter further comprises:

a support member (7) penetratingly arranged inside the tail end catheter body (3) and the tail end hard tube (4) and being made of a memory alloy material;
a contraction rope (9) disposed inside the loop of the support member (7), one end of which is fixed together with one end of the support member (7) to the atraumatic head end (8),
wherein when the contraction rope (9) contracts, the supporting member (7) is deformed so as to adjust the diameter of the annular distal end (1).

22. The catheter according to claim 21, wherein the catheter further comprises:
a protective tube (10) wrapped outside the support member (7) and the contraction rope (9).

23. The catheter according to claim 21, wherein the handle assembly (15) has a knob (17) thereon; and the knob (17) is arranged to control retraction and recovery of the contraction rope (9) by rotation.

24. The catheter according to claim 22, wherein a positioning sensor (131, 132) is fixed on the outer circumference of the protective tube (10); the positioning sensor (131, 132) is of a cylindrical structure; and the protective tube passes through the center of the cylindrical structure.

25. The catheter according to claim 24, wherein the positioning sensor (131, 132) and the protective tube (10) are secured with a sheath tube (14).

26. The catheter according to claim 20, wherein the catheter further comprises:
a push button (16) arranged between the handle assembly (15) and the proximal catheter body (6), wherein the push button (15) is connected to one end of a traction assembly (42) arranged in the distal catheter body (5), and the other end of the traction assembly (42) is connected to the tail end hard tube (4), so that the push button (16) is pushed to control the tightness of the traction assembly (42) so as to achieve the bending of the distal catheter body (5).

27. The catheter according to claim 26, wherein the traction assembly (42) is arranged sideways in the distal catheter body (5).

28. The catheter according to claim 20, wherein a positioning electrode (41) is arranged on the tail end hard tube (4) and a third positioning sensor (133) is arranged inside the tail end hard tube (4).

29. The catheter according to claim 20, wherein the connector (18, 19) comprises:

a first connector (18), connected to the positioning sensors (131, 132, 133) by a first cable, configured for transmitting positioning information; and
a second connector (19), respectively connected to the ring electrodes on each electrode pair by a second cable, configured for transmitting ablation energy.

30. A method for controlling the function of an electrode pair on an ablation catheter according to claim 1, the ablation catheter being a pulsed electric field ablation catheter for ablation of the heart and its surrounding tissues, wherein the method comprises:

in an ablation mode of the catheter, transmitting electric field energy of the same polarity to the two electrodes in each electrode pair, so that the two electrodes in the electrode pair are equivalent to the same electrode to apply a voltage to the corresponding tissue and transmit electric field energy of opposite polarity to the adjacent electrode pair, so as to realize the pulsed electric field ablation function; and
in a mapping mode of catheter, the electrophysiological signals are separately collected by two electrodes in each electrode pair, so as to realize the electrophysiological mapping function.

31. A computer-readable medium having stored thereon instructions that, when executed by a processor, cause the processor to perform the method for controlling the function of the electrode pair on the ablation catheter according to claim 30.

Fig 1

Fig 2

**Fig 3**

**Fig 4**

**Fig 5**

111

119

Fig 6

**Fig 7**

**Fig 8**

Fig 9

**Fig 10**

**Fig 11**

**Fig 12**

**Fig 13**

**Fig 14**

**Fig 15**

**Fig 16**

Fig 17

**Fig 18**

## INTERNATIONAL SEARCH REPORT

| International application No. |
| --- |
| **PCT/CN2022/128641** |

**A. CLASSIFICATION OF SUBJECT MATTER**

A61B 18/12(2006.01)i; A61B 18/14(2006.01)i; A61B 90/00(2016.01)i

According to International Patent Classification (IPC) or to both national classification and IPC

**B. FIELDS SEARCHED**

Minimum documentation searched (classification system followed by classification symbols)

A61B

Documentation searched other than minimum documentation to the extent that such documents are included in the fields searched

Electronic data base consulted during the international search (name of data base and, where practicable, search terms used)

CNTXT, ENTXTC, ENTXT, VEN, CJFD: 消融, 标测, 电生理, 弯曲, 电极, 对, 极性; ablation, mapping, electrophysiological, bent, curve, flexible, pair, electrode, polarity, negative, opposite, identical, same, positive

**C. DOCUMENTS CONSIDERED TO BE RELEVANT**

| Category* | Citation of document, with indication, where appropriate, of the relevant passages | Relevant to claim No. |
| --- | --- | --- |
| PX | CN 114366286 A (SICHUAN JINJIANG ELECTRONIC SCIENCE AND TECHNOLOGY CO., LTD.) 19 April 2022 (2022-04-19)<br>claims 1-29, 31 | 1-29, 31 |
| Y | CN 111658134 A (SICHUAN JINJIANG ELECTRONIC SCIENCE AND TECHNOLOGY CO., LTD.) 15 September 2020 (2020-09-15)<br>description paragraphs 8-41, 65-84, figures 1-19 | 1-29, 31 |
| Y | CN 113349922 A (SHANGHAI ANTAIKE MEDICAL TECHNOLOGY CO., LTD.) 07 September 2021 (2021-09-07)<br>description, paragraphs 40-55, and figures 1-2 | 1-29, 31 |
| Y | CN 112135576 A (ST JUDE MEDICAL, CARDIOLOGY DIVISION INC.) 25 December 2020 (2020-12-25)<br>description, paragraphs 29-64 | 11, 31 |
| A | CN 111772783 A (BAI, Longteng) 16 October 2020 (2020-10-16)<br>entire document | 1-29, 31 |
| A | CN 110974404 A (SICHUAN JINJIANG ELECTRONIC SCIENCE AND TECHNOLOGY CO., LTD.) 10 April 2020 (2020-04-10)<br>entire document | 1-29, 31 |

☑ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **09 January 2023** | **13 January 2023** |

| Name and mailing address of the ISA/CN | Authorized officer |
| --- | --- |
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

| International application No. |
| --- |
| **PCT/CN2022/128641** |

**C.    DOCUMENTS CONSIDERED TO BE RELEVANT**

| Category* | Citation of document, with indication, where appropriate, of the relevant passages | Relevant to claim No. |
| --- | --- | --- |
| A | US 2010191232 A1 (BOVEDA MARCO MEDICAL LLC) 29 July 2010 (2010-07-29)<br>    entire document | 1-29, 31 |
| A | WO 2021009648 A1 (CATHRX LTD.) 21 January 2021 (2021-01-21)<br>    entire document | 1-29, 31 |
| A | WO 0247569 A1 (C.R. BARD, INC. et al.) 20 June 2002 (2002-06-20)<br>    entire document | 1-29, 31 |

Form PCT/ISA/210 (second sheet) (January 2015)

## INTERNATIONAL SEARCH REPORT

| International application No. |
|---|
| **PCT/CN2022/128641** |

**Box No. II      Observations where certain claims were found unsearchable (Continuation of item 2 of first sheet)**

This international search report has not been established in respect of certain claims under Article 17(2)(a) for the following reasons:

1. ☑  Claims Nos.: **30**
   because they relate to subject matter not required to be searched by this Authority, namely:

   [1]   Claim 30 sets forth a method for controlling the function of an electrode pair on an ablation catheter, which relates to ablation interventional therapy for the human or animal body, and essentially belongs to a surgical method, and is therefore the subject matter for which a search is not required by the International Searching Authority as defined in PCT Rule 39.1(iv).

2. ☐  Claims Nos.:
   because they relate to parts of the international application that do not comply with the prescribed requirements to such an extent that no meaningful international search can be carried out, specifically:

3. ☐  Claims Nos.:
   because they are dependent claims and are not drafted in accordance with the second and third sentences of Rule 6.4(a).

Form PCT/ISA/210 (continuation of first sheet) (January 2015)

## INTERNATIONAL SEARCH REPORT
### Information on patent family members

International application No.

**PCT/CN2022/128641**

| Patent document cited in search report | | | Publication date (day/month/year) | Patent family member(s) | | | Publication date (day/month/year) |
|---|---|---|---|---|---|---|---|
| CN | 111658134 | A | 15 September 2020 | None | | | |
| CN | 113349922 | A | 07 September 2021 | None | | | |
| CN | 112135576 | A | 25 December 2020 | US | 2019350649 | A1 | 21 November 2019 |
| | | | | EP | 3768185 | A1 | 27 January 2021 |
| | | | | JP | 2021526401 | A | 07 October 2021 |
| | | | | US | 2022175445 | A1 | 09 June 2022 |
| | | | | WO | 2019226640 | A1 | 28 November 2019 |
| CN | 111772783 | A | 16 October 2020 | None | | | |
| CN | 110974404 | A | 10 April 2020 | None | | | |
| US | 2010191232 | A1 | 29 July 2010 | WO | 2010088301 | A1 | 05 August 2010 |
| WO | 2021009648 | A1 | 21 January 2021 | None | | | |
| WO | 0247569 | A1 | 20 June 2002 | AT | 333843 | T | 15 August 2006 |
| | | | | EP | 1343426 | A1 | 17 September 2003 |
| | | | | DE | 60121800 | D1 | 07 September 2006 |
| | | | | EP | 1343427 | A1 | 17 September 2003 |
| | | | | WO | 02056783 | A1 | 25 July 2002 |
| CN | 114366286 | A | 19 April 2022 | None | | | |

Form PCT/ISA/210 (patent family annex) (January 2015)